# EUROPÄISCHE PATENTSCHRIFT

(11) **EP 1 863 437 B1**
(45) Veröffentlichungstag und Bekanntmachung des Hinweises auf die Patenterteilung: **26.02.2014**
(21) Anmeldenummer: 05726197.6
(22) Anmeldetag: 24.03.2005
(51) Int. Cl.: A61Q 17/00, A61Q 19/00, A61Q 19/10, A61K 8/365, A61K 8/34, A61K 8/42

(54) **VERWENDUNG VON ZUBEREITUNGEN ZUM SCHUTZ HAUTEIGENER ENZYME**
USE OF PREPARATIONS FOR SKIN ENZYME PROTECTION
UTILISATION DE PREPARATIONS POUR LA PROTECTION DES ENZYMES CUTANEES

(43) Veröffentlichungstag der Anmeldung: 12.12.2007
(73) Patentinhaber: BEIERSDORF AG, 20245 Hamburg (DE)
(72) Erfinder: BREITENBACH, Ute, 22299 Hamburg (DE); SCHEPKY, Andreas, 25474 Bönningstedt (DE); HOLTZMANN, Ursula, 22309 Hamburg (DE); FILBRY, Alexander, 22309 Hamburg (DE)
(86) Internationale Anmeldenummer: PCT/EP2005/051394
(87) Internationale Veröffentlichungsnummer: WO 2005/063173

(56) Entgegenhaltungen:
- WO-A-99/47141
- WO-A-2005/016336
- DE-A1- 10 261 197
- US-A- 6 149 924
- US-B1- 6 312 675
- US-B1- 6 416 756
- SCHEPKY A.G., HOLTZMANN U., SIEGNER R., ZIRPINS S., SCHMUCKER R., WENCK H., WITTERN K.P., BIEL S.S.: "Influence of cleansing on stratum corneum tryptic enzyme in human skin" INTERNATIONAL JOURNAL OF COSMETIC SCIENCE, Bd. 26, 2004, Seiten 245-253, XP009057577 in der Anmeldung erwähnt

## Beschreibung

Durch äußere Einflüsse wie Umweltverschmutzung, Irritantien und Reinigung können die Haut und ihre Bestandteile wie z.B. Enzyme geschädigt und in ihrer Funktion eingeschränkt werden. Durch die Reinigung der Haut mit tensidhaltigen Formulierungen sollen Oberflächenlipide und Schmutz effektiv von der Hautoberfläche entfernt werden. Die Enzyme der Haut sollten durch diese Reinigung möglichst wenig geschädigt werden. Die üblicherweise verwendeten (anionischen) Tenside bzw. Tensidsysteme desaktivieren die Enzyme stark. Dadurch werden wichtige Stoffwechselphysiologische Prozesse (Desquamation u.ä.) der Haut nachhaltig beeinträchtigt.

Hauteigene Enzyme im Sinne der vorliegenden Schrift sind Enzyme, die auf der Hautoberfläche oder nahe der Oberflache in der Haut vorliegen. Solche Enzyme können sein: Hydrolasen, wie Proteasen, Esterasen, Lipasen, Phosphatasen, Sulfatasen und Transglutaminasen, insbesondere jedoch Proteasen wie das Stratum Comeum Tryptisches Enzym. In Tabelle 1 und 2 und im folgenden sind die wichtigsten literaturbekannten Stratum Comeum Enzyme aufgezeigt.

**Tabelle 1: Enzyme, die Desmosomen degradieren und zur Desquamation beitragen**

| Enzym | Wirkort | Reaktion (Barriereschaden) | Literatur |
|---|---|---|---|
| SCCE | SC (LB) | Spaltung von Proteinbindungen | Lundström, 1991 |
| | | | Suzuki, 1994 |
| | | | Sondell, 1995 Chang-Yi, 1997 |
| Trypsin | SC | Spaltung von Proteinbindungen ↑ | Suzuki, 1994 Chang-Yi, 1997 |
| Cathepsine | SG | Filaggrinabbau Keratinisierungshilfe | Hara, 1993 Kawada, 1997 |
| Thiol-Protease | SC | | Yokozeki, 1987 |

**Tabelle 2: Enzyme, die die Barriere aufbauen und zur Barrierehomöostase beitragen**

| Enzym | Wirkort | Reaktion (Barriereschaden) | Literatur |
|---|---|---|---|
| Phospholipase A₂ | SG-SC; LB | Freisetzung von Fettsäuren und möglicherweise Cholesterol von Cholesterotestem | Mauro, 1998 |
| | | | Mao-Qiang, 1995 |
| | | | Elias, 1988 |
| | | | Menon, 1986 |
| Saure Lipase | SC, LB | Freisetzung von Sterolen | Menon, 1986 |
| | | | Elias, 1988 |
| Neutrale Lipase | SC, LB | Sterol - und Fettsäurefreisetzung Regulation von Proteinkinasen (Differenzierung) | Menon, 1986 |
| Sphingomyelinase | SC, LB | Bereitstellung von Ceramiden | Menon, 1986 |
| Ceramidase | SC | Bereitstellung von Ceramiden | Jin, 1994 |
| ß-Glucocerebrosidase | SC | Konversion von Glyoocera-miden zu Ceramiden | Holleran, 1992 |
| | | | Mauro, 1998 |
| Steroid Sulfatase | SC | Cholesterolfreisetzung aus Cholesterolsulfat | Elias, 1988 |
| Sulfatasen | SC | Precursor-Spaltung | Baden,1980 |

Ammonia-Lyasen spielen eine wichtige Rolle beim Filaggrinabbau (Kuroda et al., 1979). Ebenso wie Transgl utaminasen (Polakowska et al., 1991), die für die Bildung des 'Cornified Envelope' essentiell sind. Phosphatasen sind die Hydrolasen mit der höchsten Gesamtaktivität im Stratum Corneum.

**Einfluß von Enzymen auf die Desquamation** (siehe Schepky et al., 2004, Influence of cleansing on stratum corneum tryptic enzyme (SCTE) in human volonteers , Int. Journal of Cosmetic Science, 26, 245 -253)

Rieger schreibt 1994 in Cosmetic & Toiletries, dass die Organisation der Epidermis eine chemische Modifikation von Bestandtelen der Keratinozyten, u.a. in den Lamellar Bodies, benötigt. Elias hat auf die Notwendigkeit von hydrolytischen (katabolen) Enzymen in der Haut hingewiesen. Proteasen werden für die Entfernung desmosomaler Strukturen benötigt . Wenn dort denaturierende Tenside eindringen und die Enzymaktivitäten stark beeinträchtigt werden, folgt daraus ein defektes Stratum Comeum .

Für den Erhalt einer konstanten Dicke des Stratum Corneums muss die Desquamationsrate und die de novo Produktion der Komeozyten exakt ausbalanciert sein. Egelrud hat den Beweis, dass die Proteolyse durch Proteasen das zentrale Ereignis im Desquamationsprozess ist, mit Hilfe eines Plantar Stratum Comeum Modells geführt. Die am besten charakterisierten Enzyme mit einer Funktion bei der Desquamation sind das Stratum Comeum Chymotryptische Enzym (SCCE) und Stratum Comeum Tryptisches Enzym (SCTE). SCCE hat einige Eigenschaften, die mit seiner Rolle bei der Desquamation in vivo gut korreilieren: das pH Profil seiner katalytischen Einheit, sein spezifisches Inhibitorprofil und seiner Lage im Gewebe. SCTE hat eine ähnliche Rolle bei der Desquamation wie SCCE, dürfte aber zusätzlich in der Lage sein, inaktives SCCE durch Hydrolyse zu aktivieren. Es wird vermutet, dass dieses Enzym sich autokatalytisch von der inaktiven in die aktive Form spaltet. Für beide Enzyme wurde gezeigt, dass eine topische Applikation von spezifischen Inhibitoren dieser Serinproteasen (Aprotinin und Leupeptin) zu mehr Hautschuppen in vivo führt. Sato et al. berichteten 1998, dass Cholesterol -3-sulfat durch kompetitive Inhibition sowohl die Aktivität von SCCE als auch von SCTE emiedrigt.

Dies geht einher mit reduzierter Desquamation . Weitere Proteasen (Cathepsin D) wurden im Stratum Corneum gefunden, sind aber wahrscheinlich hauptsächlich für die Feinjustierung des Abschilfems zuständig.

Die vorliegende Erfindung wird durch die Ansprüche definiert und betrifft einen kosmetischen Wirkkomplex zum Schutz von hauteigenen Enzymen gegen die nachteiligen Wirkungen von Reinigungsprodukten.

Folglich versteht man unter Enzymschutz im Sinne der vorliegenden Schrift eine signifikant Verminderung/Verminderung der durch Reinigung hervorgerufene Schädigung/Beeinträchtigung der beschriebenen Hautenzyme. Dies wird erfindungsgemäß erreicht durch Pflege der Haut mit Formulierungen, die Panthenol, Glycerin, Citrat (Wirkkomplex) bei pH 5 enthalten. Im Vergleich zu einem Placebo, welches den Wirkkomplex nicht enthält, wurde die Wirkung nachgewiesen.

Der Enzymschutz kann folgendermaßen quantifiziert werden: zunächst wird eine *ex vivo* Bestimmung der Wirkung von Tensiden auf die Trypsin -Aktivität in menschlicher Oberhaut durchgeführt. Probanden cremen sich drei Wochen entweder mit Placebo bzw. Verum ein und waschen sich dann ohne Pflege unter Aufsicht mehrere Male in 3 Tagen mit einem Standardduschprodukt oder Wasser auf verschiedenen Arealen. 24 h später erfolgt eine extraktion des oberen Stratum Comeums. Im Extrakt wird die Stratum corneum tryptic enzyme (SCTE) Aktivität gemessen. Parallel wird die Proteinkonzentration der Extrakte bestimmt um die spezifische Trypsinaktivität zu erhalten (Korrektur unterschiedlicher Extraktion der Areale).

Überraschend wurde gefunden, dass die Verwendung eines Wirkkomplexes, bestehend aus Panthenol, Glycerin und Citrat, bei dem das Massenverhältnis
Panthenol zu Citrat 25:1 bis 5:1, bezogen auf das Citrat-Anion beträgt, und das Massenverhältnis Panthenol zu Glycerin 1:1 bis 1:4 beträgt, zur Herstellung einer Hautpflegezubereitung, die auf einen pH-Wert von 5 eingestellt ist, zum Schutz hauteigener Enzyme vor durch spätere Reinigung hervorgerufenen Schädigungen, sowie die Verwendung eines Wirkkomplexes, bestehend aus Panthenol, Glycerin und Citrat, bei dem das Massenverhältnis
Panthenol zu Citrat 25:1 bis 5:1, bezogen auf das Citrat-Anion beträgt, und das Massenverhältnis Panthenol zu Glycerin 1:1 bis 1:4 beträgt, zur Herstellung einer Reinigungszubereitung, die auf einen pH-Wert von 5 eingestellt ist, zum Schutz hauteigener Enzyme vor durch Reinigung hervorgerufenen Schädigungen den Nachteilen des Standes der Technik abhelfen. Durch solche Zubereitungen lässt sich die durch Reinigung hervorgerufene Schädigung der Hautenzyme verringern.

Weiterhin ist es erfindungsgemäß bevorzugt, wenn das Massenverhältnis Citrat zu Glycerin 60:1 bis 10:1 beträgt, bezogen auf das Citrat -Anion. Die Erfindung umfasst auch die Verwendung des beschriebenen Wirkkomplexes in einer auf den pH-Wert 5 eingestellten Reinigungszubereitung zum Schutz hauteigener Enzyme vor durch Reinigung hervorgerufene Schädigungen.

Ebenso umfasst die Erfindung auch die Verwendung des beschriebenen Wirkkomplexes in einer auf den pH-Wert 5 eingestellten Hautpflegezubereitung zum Schutz hauteigener Enzyme vor durch spätere Reinigung hervorgerufene Schädigungen.

Auch im Rahmen eines Verfahrens zur kosmetischen Behandlung der Haut umfassend zumindest die Schritte a) topische Applikation einer Zubereitung, die den beschriebenen Wirkkomplex enthält, b) Reinigung der Haut mit einer Tenside, bevorzugt 5 bis 10% Lauryl - oder Myrethethersulfat und 2-8% Cocoamidopropylbetain enthaltenden Zubereitung ist die vorliegende Erfindung vorteilhaft einzusetzten.

Das gleiche gilt für ein Verfahren zur kosmetischen Behandlung der umfassen zumindest die Schritte a) topische Applikation einer Zubereitung, die den beschriebene Wirkkomplex enthält, b) Reinigung der Haut mit einer Tenside, bevorzugt 5 bis 10% Lauryl - oder Myrethethersulfat, 2-8% Cocoamidopropylbetain und 1 -5 % eines weiteren Cotensides enthaltenden Zubereitung.

Darüber hinaus können erfindungsgemäße Zubereitungen außerdem Substanzen enthalten, die UV-Strahlung im UVB-Bereich absorbieren, wobei die Ge samtmenge der Filtersubstanzen z.B. 0,1 Gew. -% bis 30 Gew.-%, vorzugsweise 0,5 bis 10 Gew. -%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Son nenschutzmittel fürs Haar dienen.

Vorteilhafte UV-A-Filtersubstanzen im Sinne der vorliegenden Erfindung sind Di benzoylmethanderivate, insbesondere das 4 -(tert.-Butyl)-4'-methoxydibenzoylmethan (CAS-Nr. 70356-09-1), welches von Givaudan unter der Marke Parsol ^{®} 1789 und von Merck unter der Handelsbezeichnung Eusolex® 9020 verkauft wird.

Vorteilhafte weitere UV-Filtersubstanzen im Sinne der vorliegenden Erfindung sind sulfo - nierte, wasserlösliche UV -Filter, wie z. B.:
- Phenylen-1,4-bis-(2-benzimidazyl)-3,3'-5,5'-tetrasulfonsäure und ihre Salze, besonders die entsprechenden Natrium -, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol -5-sulfonsäure, wie ihr Natrium -, Kalium- oder ihr Triethanolammonium-Salz sowie die Sulfonsäure selbst mit der INCI Bezeichnung Phe nylbenzimidazole Sulfonsäure (CAS. -Nr. 27503-81-7), welches beispielsweise unter der Handelsbezeichnung Eusolex 232 bei Merck oder unter Neo Heliopan Hydro bei Haar - mann & Reimer erhäft lich ist;

Vorteilhafte UV-Filtersubstanzen im Sinne der vorliegenden Erfindung sind ferner sogenannte Breitbandfilter, d.h. Filtersubstanzen, die sowohl UV -A- als auch UV-B-Strahlung absorbieren.

Vorteilhafte Breitbandfilter oder UV -B-Filtersubstanzen sind beispielsweise Triazinderivate, wie z. B.
- 2,4-Bis-{[4-(2-Ethyl-hexyloxy)-2-hydroxy]henyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- 4,4,4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Tria-zone), welches von der BASF Aktien gesellschaft unter der Warenbezeichnung UVINUL® T 150 vertrie ben wird.

Die weiteren UV-Filtersubstanzen können öllöslich oder wasserlöslich sein.

Vorteilhafte öllösliche UV -B- und/oder Breitband -Filtersubstanzen im Sinne der vorliegenden Erfindung sind z. B.:
■ Zimtsäurederivate, vorzugsweise 2-Ethylhexylmethoxycinnamate (CAS-Nr.: 5466-77-3), welches unter der Handelsbezeichnung Parsol MCX bei der Firma Givaudan erhältlich ist.

Die Liste der genannten UV -Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV -strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

### Beispiele

### 1) Bestimmung ex vivo der Wirkung von Tensiden auf die Trypsin -Aktivität in menschlicher Oberhaut

Für die Standardisierung der Probandenhaut wurden die Probanden gebeten, drei Wochen nur ein mildes Duschgel (6% Natrium Myrethsulfat, 8% Natrium Cocoamphoacetat ) beim Waschen zu benutzen. Parallel cremten sich die Probanden zwei Mal täglich drei Wochen einen Unterarm mit Beispiel 1 versus den anderen Unterarm mit Beispielen 2, 3, 4 oder 5 ein. Die Unterarme wurden jeweils in zwei Testarealen geteilt. Die Pflege wurde direkt vor Beginn des Waschprozesses beendet. Die Testareale wurden drei Tage nacheinander jeweils 3 mal täglich mit 1 mL Waschprodukt 45 s behandelt. Nach der Behandlung wurde das Testareal mit Leitungswasser 30 s abgespült und mit einem Einweg Papiertuch abgetrocknet. Am 1. und 2. Tag wurden die Areale drei Mal behandelt (morgens, mittags und nachmittags), am 3. Tag wurden sie zwei Mal behandelt (morgens und mittags).

### 2) Extraktion der Haut Biopsie und Messung der SCTE -Aktivität

Am 4. Tag wurden SC -Proben aus den Arealen mittels eines mit Zuckerlösung beschichteten Objektträgers gestrippt. Später wurden die Komeozyten vom Objektträger mit PBS Puffer-Lösung abgelöst und die spezifische SCTE -Aktivität wurde bestimmt

### 3) Stratum corneum tryptic enzyme (SCTE) activity assay

100 µl Humanhautextrakt wurden 24h mit 150 µL N-t BOC-Phe-Ser-Arg-7-amido-4-methylcoumarin (33 µM in PBS; Sigma, St. Louis, USA) bei 37°C inkubiert. Die SCTE - specifische Freisetzung von fluoreszierendem 7 -amino-4-methylcoumarin wurde mittels eines Fluoreszenzplattenreaders (Filter ex = 360 nm ± 40, em = 460 nm ± 40 nm, CytoFluor 4000, PerSeptive Blosystems, Framingham, USA) ermittelt.

### 4) Messung der Proteinkonzentration

Um die spezifische Trypsinaktivität der Extrakte auszurechnen, wurde der Proteingehalt mittels der Ninhydrin-Methode nach alkalischer Hydrolyse bestimmt. Die Komeozyten-Lösungen wurde zur Trockne eingeengt und die Proteine wurden 5 h bei 150°C mit 2 mL Natronlauge (6M) hydrolysiert. Die Lösung wurde mit 2 mL Salzsäure (6M) neutralisiert und 1 mL Natrium Propionsäure -Puffer (3,35 M, pH 5,5) wurde zugegeben. Danach wurden 50 µL des Lysats mit 450 uL Bidest. -Wasser verdünnt und 20 min bei 70°C mit 25 µL Ameisensäure (0,4% (v/v)) und 500 µL Ninhydrin-Lösung (2 % (w/v) Ninhydrin in 3,35 M Natrium Propionsäure-Puffer mit 50% (v/v) Ethylenglykolmon omethylether (Sigma, St. Louis, USA) inkubiert. Nach Abkühlen wurden 5 mL Ethanol (50 % (v/v) in bidest -Wasser) zugegeben. Die Absorption wurde bei einer Wellenlänge von 570 nm mit einem Spectrophotometer gemessen (UVICON 942, Kontron, Mitano, Italy) und die entsprechende Proteinkonzentration wurde berechnet.

### 5) Formeln

| **Beispiel Nr.** | **1 Placebo** | **2** | **3** | **4** | **5** |
|---|---|---|---|---|---|
| **Cetearyl Alcohol + PEG -40 Castor Oil + Sodium Cetearyl Sulfate** | | | **2** | **1** | |
| **Ceteth-20 + Glyceryl Stearate** | | | **1,3** | | |
| **Lanolin Alcohol** | | | **0,2** | | |
| **Polyglyceryl-2 Dipolyhydroxystearate** | | | | | **3** |
| **Polyglyceryl-3 Diisostearate** | | | | | **2** |
| **Polyglyceryl-3 Methylglucose Distearate** | **5** | **5** | | | |
| **Sorbitan Stearate** | **2** | **2** | **1** | **2** | |
| **Diazolidinyl Urea** | **0,25** | **0,25** | | | |
| **Phenoxyethanol + Methylparaben + Ethylparaben + Butylparaben + Isobutylparaben + Propylparaben** | **0,5** | **0,5** | **0,5** | **0,5** | **0,5** |
| **Cera Microcristal lina** | | | **5** | | |
| **Paraffinum Liquidum** | | | | **8** | **10** |
| **Glycerin** | | **5** | **5** | **12** | **10** |
| **Cetearyl Ethylhexanoate** | | | **0,8** | | |
| **Isopropyl Myristate** | | | **5** | | |
| **Isopropyl Stearate** | | | | | **9** |
| **Parfum** | **0,15** | **0,15** | **0,15** | **0,15** | **0,15** |
| **Citric Acid** | | **0,1** | **0,1** | **0,1** | **0,1** |
| **Diammonium Citrate** | | | **0,25** | | |
| **Sodium Citrate** | | **0,174** | | **0,174** | **0,174** |
| **Magnesium Sulfate** | | | | | **0,6** |
| **Cyclomethicone** | **5** | **5** | | **3** | |
| **Carbomer** | | | | **0,25** | |
| **Sodium Hydroxide** | | | | **0,03** | |
| **Cetearyl Alcohol** | **2** | **2** | | | |
| **Cetyl Alcohol** | | | **2,5** | **3** | |
| **Cetyl Palmitate** | | | **10** | **10** | |
| **PEG-150 Distearate** | **1** | **1** | | | |
| **Panthenol** | | **3** | **3** | **5** | **3** |
| **Aqua** | **ad 100** | **ad 100** | **ad 100** | **ad 100** | **ad 100** |
| | | | | | |
| **pH - Wert** | **7** | **5** | **5** | **5** | **5** |
| SCTE-Wert normiert auf Placebo =100 | 100 | 155 | 156 | 148 | 125 |

## Patentansprüche

1. Verwendung eines Wirkkomplexes, bestehend aus Panthenol, Glycerin und Citrat, bei dem das Massenverhältnis
Panthenol zu Citrat 25:1 bis 5:1, bezogen auf das Citrat-Anion beträgt, und das Massenverhältnis Panthenol zu Glycerin 1:1 bis 1:4 beträgt, zur Herstellung einer Reinigungszubereitung, die auf einen pH-Wert von 5 eingestellt ist, zum Schutz hauteigener Enzyme vor durch Reinigung hervorgerufenen Schädigungen.

2. Verwendung eines Wirkkomplexes, bestehend aus Panthenol, Glycerin und Citrat, bei dem das Massenverhältnis
Panthenol zu Citrat 25:1 bis 5:1, bezogen auf das Citrat-Anion beträgt, und das Massenverhältnis Panthenol zu Glycerin 1:1 bis 1:4 beträgt, zur Herstellung einer Hautpflegezubereitung, die auf einen pH-Wert von 5 eingestellt ist, zum Schutz hauteigener Enzyme vor durch spätere Reinigung hervorgerufenen Schädigungen.

## Claims

1. Use of an active complex consisting of panthenol, glycerine, and citrate, wherein the weight ratio Panthenol to citrate is from 25: 1 to 5:1, related to the citrate anion, and the weight ratio Panthenol to glycerol is from 1: 1 to 1 : 4 for preparing a cleansing composition, which is adjusted to a pH of 5, in order to protect the skin enzymes against damage caused by cleansing.

2. Use of an active complex consisting of panthenol, glycerine, and citrate, wherein the weight ratio of Panthenol to citrate is from 25: 1 to 5:1, related to the citrate anion, and the weight ratio of Panthenol to glycerol is from 1: 1 to 1 : 4 for preparing a skin care composition, which is adjusted to a pH of 5, in order to protect the skin enzymes against damage caused by later cleansing.

## Revendications

1. Utilisation d'un complexe actif constitué par le panthénol, la glycérine, et du citrate, dans laquelle le rapport du poids du panthénol au citrate est de 25: 1 à 5:1, par rapport à l'anion citrate et le rapport en poids du panthénol à la glycérine est de 1: 1 à 1: 4 pour la préparation d'une composition de nettoyage, qui est ajusté à un pH de 5, afin de protéger les enzymes de la peau contre les dommages causés par le nettoyage.

2. Utilisation d'un complexe actif constitué par le panthénol, la glycérine, et du citrate, dans laquelle le rapport du poids du panthénol au citrate est de 25: 1 à 5:1, par rapport à l'anion citrate et le rapport en poids du panthénol à la glycérine est de 1: 1 à 1: 4 pour la préparation d'une composition de soin de la peau, qui est ajusté à un pH de 5, afin de protéger les enzymes de la peau contre les dommages causés par un nettoyage subséquent.
